# EUROPÄISCHE PATENTSCHRIFT

(11) **EP 1 802 259 B1**
(45) Veröffentlichungstag und Bekanntmachung des Hinweises auf die Patenterteilung: **05.03.2008**
(21) Anmeldenummer: 05791405.3
(22) Anmeldetag: 18.10.2005
(51) Int. Cl.: A61F 13/02, A61K 9/00

(54) **Selbstschliessendes antiseptisches Pflaster**
Self-sealing antiseptic plaster
Pansement antiséptique auto-obturant

(30) Priorität: 20.10.2004 CH 17292004
(43) Veröffentlichungstag der Anmeldung: 04.07.2007
(73) Patentinhaber: Vostra-Med AG, 6330 Cham (CH)
(72) Erfinder: HARREN, Ernst-Diethelm, CH-6312 Steinhausen (CH); LEHMANN, Marc, Ulrich, CH-8704 Herrliberg (CH)
(74) Vertreter: EGLI-EUROPEAN PATENT ATTORNEYS
(86) Internationale Anmeldenummer: PCT/CH2005/000607
(87) Internationale Veröffentlichungsnummer: WO 2006/042429

(56) Entgegenhaltungen:
- EP-A- 0 368 264
- EP-A- 0 424 165
- US-A- 5 015 228
- US-A- 5 330 452

## Beschreibung

Die Erfindung betrifft ein selbstschliessendes antiseptisches Pflaster nach Patentanspruch 1. Es ist für die Verwendung am menschlichen oder tierischen Körper vorgesehen.

Die Erfindung betrifft insbesondere ein selbstschliessendes antiseptisches Pflaster für die Punktierung von Blutgefässen, von Muskelgewebe, von Gewebe, von Haut, von Organen (Biopsie) und von Knochenmark, das von Kanülen oder Nadeln oder dergleichen durchstechbar und mindestens in einem Teilbereich transparent ist.

In diesem Bereich der medizinischen Anwendungen gibt es einen Bedarf, die Einstichstelle und den Bereich in unmittelbarer Umgebung der Einstichstelle vor, während und nach dem Einstich möglichst einfach und zuverlässig antiseptisch zu versiegeln und damit diesen Zustand über einen definierbaren Zeitraum zu erhalten.

In herkömmlicher Weise geschieht dies, indem die zu punktierende Stelle beispielsweise zunächst mit einem Desinfektionsmittel im Sprühverfahren oder dergleichen desinfiziert wird, dann der Einstich und der damit verbundene medizinische, invasive Vorgang mit einer sterilen Nadel oder Kanüle oder dergleichen durchgeführt wird, und schliesslich die Einstichstelle zwecks Stillung der Blutung mit einem geeigneten Mittel, wie beispielsweise Pflaster oder Verband, abgedeckt wird.

Die US-5 015 228 beschreibt ein desinfizierend wirkendes Pflaster, das bereits vor dem Einstich an der betreffenden Stelle auf der Haut des Patienten aufgeklebt wird. Es handelt sich also um ein Pflaster, das durchstechbar ist und das zu diesem Zweck im dafür vorgesehenen Bereich transparent ist.

Dieses Pflaster hat eine Trägerschicht, die in einem Teilbereich eine Ausnehmung aufweist. Auf der Trägerschicht und über der Ausnehmung ist eine transparente, durchstechbare und sich selbst wieder verschliessenden Verschlussschicht mit desinfizierenden Eigenschaften angebracht.. Die Unterseite der Trägerschicht ist mit einer Hautkleberschicht versehen, also mit einem Kleber, der gute Hautverträglichkeit aufweist. Als Verschlussschicht wird bei diesem Pflaster ein Gel, das mit desinfizierenden Substanzen durchsetzt ist, verwendet. Dabei können die desinfizierenden Substanzen anti-bakterielle, anti-virale oder anti-fungizide Eigenschaften oder eine Kombination dieser Eigenschaften aufweisen.

Das Pflaster der US-5 015 228 soll es ermöglichen, dass die zu punktierende Stelle nicht mehr vorgängig separat desinfiziert werden muss, indem es nämlich direkt auf die betreffende Stelle aufgeklebt wird. Das zu punktierende Gefäss ist durch die Ausnehmung in der Trägerschicht und durch die Verschlussschicht mit Gel-Medium sichtbar und ertastbar. Somit kommt die Haut des Patienten beim Aufbringen des Pflasters mit der Hautkleberschicht und der desinfizierend wirkenden Verschlussschicht in Berührung. Beim Einstich durch die Verschlussschicht wird die Nadel desinfiziert und beim Herausziehen der Nadel verschliesst sich die Verschlussschicht mit dem Gel-Medium dank deren Wiederverschliess-Eigenschaften sofort wieder. Das Pflaster kann offenbar nach dem Einstich auf der Einstichwunde verbleiben und soll so vor, während und nach dem medizinischen Vorgang den sterilen Zustand im Bereich der Einstichstelle aufrechterhalten.

Bei dieser Lösung besteht jedoch die Gefahr, dass ein Blutgefäss durch die Ausnehmung und durch die Verschlussschicht mit dem Gel-Medium nur ungenügend sichtbar und ertastbar ist, weil nur eine relativ kleine Ausnehmung in einer ansonsten undurchsichtigen Trägerschicht vorhanden ist. Ebenso besteht die Gefahr, dass die Einstichstelle und der Bereich um die Einstichstelle vor dem Einstich möglicherweise nur ungenügend desinfiziert werden. Da zudem an der Einstichstelle selber nur die dünne elastische Verschlussschicht mit dem Gel-Medium wirksam ist, besteht die erhöhte Gefahr, dass ein Blutaustritt nach dem Herausziehen der Nadel erfolgen kann und somit zusätzliche Massnahmen zur Blutstillung erforderlich werden.

Aufgabe der Erfindung ist es deshalb, ein verbessertes selbstschliessendes antiseptisches.Pflaster anzugeben.

Die antiseptische Wirkung des Pflasters soll dabei darin bestehen, dass die Einstichstelle und der Bereich in der unmittelbaren Umgebung der Einstichstelle vor, während und nach dem Einstich möglichst einfach und zuverlässig antiseptisch erhalten wird. Zusätzlich soll der Blutaustritt nach dem Herausziehen der Nadel aus der Einstichwunde möglichst vermieden werden, um so das ärztliche- und Pflegepersonal vor möglichen Infektionen zu schützen und die allenfalls erforderliche anschliessende Stillung der Blutung zu erleichtern.

Diese Aufgabe wird durch die Merkmale des Patentanspruchs 1 gelöst.

Die Lösung besteht darin, dass ein selbstschliessendes antiseptisches Pflaster auf die vorher gereinigte und desinfizierte Körperstelle aufgeklebt wird und so die zu punktierende Stelle in ihrem antiseptischen Zustand versiegelt. Das selbstschliessende antiseptische Pflaster weist eine durchgehende Trägerschicht ohne Ausnehmungen auf. Dabei ist die gesamte Trägerschicht des Pflasters auf derjenigen Seite, die mit der Haut in Berührung kommt, mit einer Hautkleberschicht versehen. Auf der Trägerschicht ist mittels einer Kleberschicht eine Verschlussschicht mit selbstverschliessenden Eigenschaften angebracht. Zudem sind die Trägerschicht, die Verschlussschicht, die Kleberschicht und die Hautkleberschicht vorzugsweise im gesamten Bereich des selbstschliessenden antiseptischen Pflasters oder zumindest in einem übereinanderliegenden Teilbereich desselben transparent oder annähernd transparent. Das erfindungsgemässe selbstschliessende antiseptische Pflaster kann somit nach erfolgtem Eingriff ebenfalls auf der Punktionsstelle belassen werden und sorgt so für einen anhaltenden antiseptischen Zustand.

Durch die Einführung einer Trägerschicht und einer darauf mittels einer Kleberschicht angebrachten Verschlussschicht mit selbstverschliessenden Eigenschaften wird erreicht, dass für die Verschlussschicht eine Vielzahl von geeigneten Materialien mit selbstverschliessenden Eigenschaften verwendbar werden, ohne dass man gleichzeitig wegen fehlenden, dazu passenden und geeigneten Hautkleberschichten, wie z.B. solchen, die mit antiallergischen, antiseptischen oder auch analgetischen Wirkstoffen angereichert sind, an deren Einsatz gehindert ist. Die Trägerschicht dient somit einer gewissen ,Entkopplung' der Funktionen und sie dient auch einer Stabilisierung der mechanischen Eigenschaften der Verschlussschicht, z.B. der Dehnbarkeit, die bei gewissen Materialien mit günstigen selbstverschliessenden Eigenschaften, wie z.B. Silikon, notwendig und erwünscht ist.

Die Trägerschicht und auch die Verschlussschicht sind zudem vorteilhafterweise auch wasserdampfdurchlässig ausgebildet, um den Feuchtigkeitstransport von der Haut an die Umgebung möglichst zu erleichtern. Die Trägerschicht kann auch flüssigkeitsabsorbierende Eigenschaften aufweisen.

Durch die Ausdehnung der Trägerschicht und der darauf angebrachten Verschlussschicht auf den gesamten Bereich des selbstschliessenden antiseptischen Pflasters, sowie der Transparenz aller vorhandener Schichten, wird erreicht, dass die Platzierung und der Gebrauch des selbstschliessenden antiseptischen Pflasters wesentlich erleichtert werden. Die Sichtbarkeit der zu punktierenden Stelle und vor allem auch die Ertastbarkeit von Blutgefässen sind wesentlich verbessert gegenüber einer Lösung mit Ausnehmungen. Der besseren Ertastbarkeit von Blutgefässen dient natürlich auch, dass sowohl die Trägerschicht als auch die Verschlussschicht aus dünnen und weichen Materialien gefertigt sind. Im Weiteren wird damit aber auch erreicht, dass die Einstichstelle selber wie mit einer Art zusätzlichen ,künstlichen Haut' so fest, elastisch und nach dem Herausziehen der Kanüle oder Nadel aus der Einstichstelle selbstverschliessend verschlossen wird, dass aus dem Einstichkanal im Normalfall kein Blut austreten kann.

Vorzugsweise wird als Material für die Verschlussschicht für medizinische Zwecke geeignetes Silikon verwendet. Es hat nicht nur hervorragende Eigenschaften bezüglich Verträglichkeit mit dem menschlichen oder tierischen Körper, sondern insbesondere auch bezüglich der Dehnbarkeit bzw. der elastischen Rückstellkraft. Diese letztere Eigenschaft bewirkt denn auch, dass ein von einer Kanüle oder Nadel geschaffener Einstichkanal sich nach dem Zurückziehen der Nadel sofort und selbsttätig wieder so gut verschliesst, dass kein Blut austritt. Sie bewirkt zugleich auch, dass das Material beim Einstich auf den ersten Millimetern zwar geschnitten, dann aber auseinandergeschoben bzw. verdrängt wird, so dass das Ausstanzen bzw. Heraustrennen von Materialpartikeln, die sonst in den Körper des Patienten gelangen könnten, zuverlässig vermieden wird.

Silikon ist andererseits aber auch ein Material, das nicht problemlos mit allen anderen Materialien verklebbar ist. Insbesondere das zuverlässige direkte und dauerhafte Aufbringen eines Klebers, der zusätzlich auch noch die gewünschten Eigenschaften bezüglich Hautverträglichkeit aufweist, ist bisher noch nicht zufriedenstellend gelöst. Deshalb, und natürlich auch mit Hinblick auf die erwünschte Stabilisierwirkung wegen der ausserordentlich hohen Dehnfähigkeit von Silikon, erweist sich die verwendete Trägerschicht als doppelt nützlich. Der erfindungsgemäss zwischen der Verschlussschicht und der Trägerschicht verwendete Kleber braucht also keine hautkleberspezifischen Eigenschaften aufzuweisen sondern muss lediglich für medizinische Anwendungen zugelassen sein.

Es kann auch noch vorgesehen sein, dass in der Kleberschicht zwischen der Verschlusschicht und der Trägerschicht in einem für Nadeleinstiche vorgesehenen Bereich eine kleberfreie Aussparung vorhanden ist. Damit wird erreicht, dass selbst beim Einstich mit einer Nadel oder Kanüle mit Sicherheit keine Spuren von Materialpartikeln des Klebers in den Körper gelangen können.

Des weiteren kann auch vorgesehen sein, dass die Kleberschicht, die im einfachsten Fall nur aus einem Kleber (auch als technischer Kleber bezeichnet, d.h. ein Kleber ohne hautkleberspezifische Eigenschaften) besteht, einen Trägerfilm aufweist, der beidseitig mit Kleber versehen ist. Ein solcher Aufbau der Kleberschicht kann gewählt werden, um die Produktion in dem Sinne zu vereinfachen, dass sie als 'Zulieferteil' für die Herstellung des selbstschliessenden antiseptischen Pflasters eingesetzt werden kann.

Da die Trägerschicht selbst auch eine gewisse, allerdings geringere, Dehnfähigkeit besitzt, wird die ausserordentlich hohe Dehnfähigkeit von Silikon durch die Trägerschicht in der flächigen Ausdehnbarkeit begrenzt und stabilisiert. Damit wird auf die Einstichwunde zum Zweck der Blutstillung eine gewisse Druckwirkung ausgeübt.

Um den dauerhaften Kontakt von Silikon mit dem verwendeten Kleber zu verbessern kann auch eine Oberflächenbehandlung des Silikons, wie z.B. eine Plasma-, corona-, nasschemische-, oder sonstige Behandlung vorgesehen sein.

Selbstverständlich können für die Verschlussschicht auch andere Materialien mit selbstverschliessenden Eigenschaften bzw. einer hohen elastischen Rückstellkraft, wie beispielsweise Naturkautschuk, Kunstkautschuk, Gummi, Latex, Hydrogel, polymerem Kunststoff eingesetzt werden. Ebenso sind Kombinationen dieser Materialien, auch mit Silikon, einsetzbar.

Die Gesamtdicke eines erfindungsgemässen selbstschliessenden antiseptischen Pflasters bewegt sich vorzugsweise etwa im Bereich von 1.0 bis 5 mm.

Zum Zwecke des Schutzes des Hautklebers ist das selbstschliessende antiseptische Pflaster hautkleberseitig mit abziehbaren Schutzfolien versehen.

Das erfindungsgemässe selbstschliessende antiseptische Pflaster kann selbstverständlich je nach medizinischem Verwendungszweck in geeigneten Grössen und Geometrien hergestellt werden. Da es jedoch bevorzugt flächig ausgestaltet ist, ist es sowohl in der Herstellung als auch im Zuschnitt besonders einfach zu handhaben. Es können aber auch nicht-flächenförmige, wie beispielsweise leicht linsenförmige, Ausgestaltungen vorgesehen sein.

Im folgenden wird das erfindungsgemässe selbstschliessende antiseptische Pflaster anhand eines Ausführungsbeispieles mit Zeichnungen detailliert beschrieben.

In den Zeichnungen zeigen:
- Fig. 1: einen Querschnitt durch ein selbstschliessendes antiseptisches Pflaster, und
- Fig. 2: einen Querschnitt durch ein selbstschliessendes antiseptisches Pflaster gemäss Fig. 1 mit abziehbaren Schutzfolien.

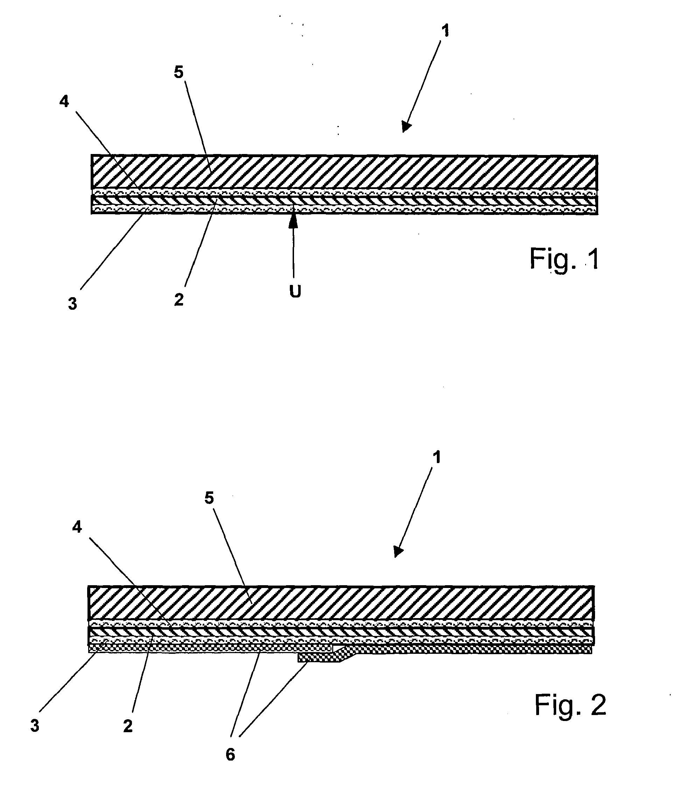

Die Figur 1 zeigt einen nicht-massstäblichen Querschnitt durch ein selbstschliessendes antiseptisches Pflaster 1. Eine in der Regel dünne Trägerschicht 2 ist beidseitig mit Klebstoffschichten versehen. An einer Unterseite U der Trägerschicht 2 befindet sich eine (medizinische) Hautkleberschicht 3, wobei diese Seite des selbstschliessenden antiseptischen Pflasters 1 zum Aufkleben auf die menschliche Haut vorgesehen ist. Auf der gegenüberliegenden Seite der Trägerschicht 2 befindet sich eine (technische) Kleberschicht 4. Die Kleberschicht 4 befindet sich zwischen der Trägerschicht 2 und einer Verschlussschicht 5 und hat keine hautkleberspezifischen Eigenschaften. Da für die Verschlussschicht 5 Materialien wie beispielsweise Silikon verwendet werden, für die nur Kleber bekannt sind, die keine oder keine besonders gute Hautverträglichkeit aufweisen, braucht bei diesem Aufbau des selbstschliessenden antiseptischen Pflasters 1 die Kleberschicht 4 keine hautkleberspezifischen bzw. besonders hautfreundlichen Eigenschaften aufzuweisen, da sie keinen Hautkontakt hat. Der Kleber für die Kleberschicht 4 muss lediglich für medizinische Zwecke geeignet sein.

Alle verwendeten Schichten, d.h. die Trägerschicht 2, die Verschlussschicht 5, die Kleberschicht 4 und die Hautkleberschicht 3 sind vorzugsweise im ganzen Bereich des selbstschliessenden antiseptischen Pflasters 1 oder aber zumindest in einem genügend grossen übereinanderliegenden Teilbereich desselben transparent oder annähernd transparent. Zudem sind für die Trägerschicht 2 und die Verschlussschicht 5 dünne und weiche Materialien vorgesehen, damit die Punktionsstelle durch das selbstschliessende antiseptische Pflaster sichtbar und gut ertastbar ist.

Die Trägerschicht 2 ist vorzugsweise aus einem Material, das weniger dehnbar ist als die Verschlussschicht 5. Damit wird die höhere Dehnfähigkeit der Verschlussschicht 5 in der flächigen Ausdehnung begrenzt und stabilisiert. Wird für die Kleberschicht 4 ein Schichtaufbau mit einem Trägerfilm und beidseitig auf dem Trägerfilm aufgebrachtem (technischen) Kleber verwendet, so ist natürlich zu erwarten, dass dieser Trägerfilm, ebenso wie die Trägerschicht 2, und natürlich auch in Abhängigkeit von der Materialwahl, zur Ausdehnungsbegrenzung und Stabilisierung der Verschlusschicht 5 beiträgt.

Die Trägerschicht 2 ist in der Regel dünn, vorzugsweise ausserhalb eines Durchstechbereiches wasserdampfdurchlässig und weist eine Dicke im Bereich von 0.01 bis ca. 1 mm auf. Um Wasserdampfdurchlässigkeit oder auch Absorptionsfähigkeit zu erreichen können Materialien wie Vliessstoff oder Papier eingesetzt werden.

In einer weiteren Ausführungsform besteht die Trägerschicht 2 beispielsweise aus Polyurethan, ist sehr dünn (folienartig) und weist eine Dicke im Bereich von 0.01 - 0.05 mm auf. Es können aber auch andere Materialien wie Polyethylen verwendet werden.

Die Verschlussschicht 5 besteht vorzugsweise aus Silikon und weist eine Dicke im Bereich von 1 bis 5 mm auf. Es können aber auch andere Materialien wie Naturkautschuk, Kunstkautschuk, Gummi, Latex, Hydrogel, polymerer Kunststoff oder einer Kombination dieser Materialien verwendet werden die selbstverschliessende Eigenschaften aufweisen.

Weiterhin wird angenommen, dass die verwendbaren Klebstoffe, d.h. solche mit geeigneten Eigenschaften für die Kleberschicht 4 und die Hautkleberschicht 3, dem Fachmann hinlänglich bekannt sind. Insbesondere bei der Hautkleberschicht ist davon auszugehen, dass in der Regel ein Klebstoff mit einer oder mehreren Eigenschaften aus der folgenden Gruppe von Eigenschaften durch Beigabe entsprechender Wirkstoffe gewählt werden muss:
- antiseptische Eigenschaften,
- antiallergische Eigenschaften,
- analgetische Eigenschaften, etc.

Wird Silikon als Verschlussschicht 5 verwendet, so kann es notwendig sein, die Kontaktfläche des Silikons mit der Kleberschicht 4 in geeigneter Weise vorzubereiten. Um den dauerhaften Kontakt von Silikon mit dem verwendeten Kleber zu verbessern kann deshalb eine Oberflächenbehandlung wie z.B. eine Plasma-, corona-, nasschemische-, oder sonstige Behandlung vorgesehen sein.

Die Figur 2 zeigt schliesslich noch einen Querschnitt durch ein selbstschliessendes antiseptisches Pflaster gemäss Fig. 1 mit abziehbaren Schutzfolien 6.

Zur Verwendung des selbstschliessenden antiseptischen Pflasters:
- Die Punktionsstelle wird lokalisiert.
- Die zu punktierende Körperstelle wird in herkömmlicher Weise gereinigt und desinfiziert.
- Beim selbstschliessenden antiseptischen Pflaster 1 werden die Schutzfolien 6 abgezogen und das Pflaster 1 wird mit der Unterseite U der Trägerschicht 2, auf welcher die Hautkleberschicht 3 aufgebracht ist, auf die betreffende Körperstelle aufgeklebt. Damit wird der antiseptische Zustand vor der Punktierung konserviert.
- Für die Punktierung eines Gefässes, von Gewebe, von Haut, von Organen (Biopsie) oder von Knochenmark wird die Stelle visuell und/oder durch Ertasten durch die transparente Oberfläche des selbstschliessenden antiseptischen Pflasters erneut lokalisiert.
- Die Punktierung wird durchgeführt, indem das selbstschliessende antiseptische Pflaster bzw. alle Schichten desselben mit einer Nadel oder Kanüle oder dergleichen durchstochen wird. Dem zu behandelnden Patienten wird die vorgesehene Menge Flüssigkeit und/oder Gewebe durch die Nadel oder Kanüle entnommen oder nur Flüssigkeit zugeführt. Da stets davon ausgegangen wird, dass der Eingriff von medizinischem Fachpersonal durchgeführt wird, wird auch vorausgesetzt, dass die verwendete Spritze oder Kanüle bereits steril ist. Der antiseptische Zustand wird somit auch während der Punktierung erhalten. Wegen der elastischen Rückstellkraft der Verschlussschicht 5 schützt dieselbe die zu punktierende Stelle auch während des Eingriffes vor Umwelteinflüssen, weil das Material stets dicht an der Nadel oder Kanüle anliegt. Ausserdem wird das Personal vor möglichen Infektionen geschützt.
- Nach erfolgtem Eingriff wird die Nadel oder Kanüle herausgezogen. Abermals wirkt die elastische Rückstellkraft der Verschlussschicht 5 in einer Weise, dass der Einstichkanal durch die Rückstellkraft der Verschlussschicht sofort wieder verschlossen wird. Somit wird die punktierte Stelle auch nach dem Eingriff zuverlässig geschützt. Es können keine Keime, mikroskopisch und/oder makroskopische Schmutzpartikel und/oder Viren und/oder Bakterien in den Einstichkanal und somit in den menschlichen oder tierischen Körper gelangen.
   - Durch die beschriebene Bauweise des selbstschliessenden antiseptischen Pflasters wird der Blutaustritt aus der Einstichwunde im Normalfall ausgeschlossen. Bei Punktierungen von Blutgefässen kann unter Umständen auch der Patient, soweit er körperlich oder geistig in der Lage ist, die Einstichstelle durch Druck auf das selbstschliessende antiseptische Pflaster zusätzlich solange komprimieren, bis sich die Einstichstelle durch die Gerinnung des Blutes selbstständig verschlossen hat. Das selbstschliessende antiseptische Pflaster kann jedenfalls so lange wie notwendig oder wünschenswert auf der betreffenden Körperstelle verbleiben.

### Bezugsziffernliste

- 1: Pflaster
- 2: Trägerschicht
- 3: (Medizinische) Hautkleberschicht
- 4: (Technische) Kleberschicht
- 5: Verschlussschicht
- 6: Schutzfolie

- U: Unterseite der Trägerschicht

## Patentansprüche

1. Selbstschliessendes antiseptisches Pflaster (1) für die Punktierung von Blutgefässen, von Muskelgewebe, von Gewebe, von Haut, von Organen und von Knochenmark, das von Kanülen, Nadeln oder dergleichen durchstechbar und in einem Teilbereich transparent ist, mit
- einer Trägerschicht (2),
- einer auf der Trägerschicht (2) angebrachten transparenten und durchstechbaren Verschlussschicht (5) aus einem Material mit elastischer Rückstellkraft,
- einer an der Unterseite U der Trägerschicht (2) angebrachten Hautkleberschicht (3),
**dadurch gekennzeichnet, dass**
- die Trägerschicht (2) keine Ausnehmungen aufweist,
- zwischen der Trägerschicht (2) und der Verschlussschicht (5) eine Kleberschicht (4) vorhanden ist, und
- die Trägerschicht (2), die Verschlussschicht (5), die Kleberschicht (4) und die Hautkleberschicht (3) im ganzen Bereich oder zumindest in einem übereinanderliegenden Teilbereich transparent oder annähernd transparent sind.

2. Selbstschliessendes antiseptisches Pflaster (1) nach Patentanspruch 1, **dadurch gekennzeichnet, dass** die Trägerschicht (2) und die Verschlussschicht (5) aus dünnen und weichen Materialien gefertigt sind, damit die zu punktierende Stelle durch das selbstschliessende antiseptische Pflaster (1) ertastbar ist.

3. Selbstschliessendes antiseptisches Pflaster (1) nach Patentanspruch 1 oder 2, **dadurch gekennzeichnet, dass** die Trägerschicht (2) und/oder die Verschlussschicht (5) ausserhalb eines Durchstechbereiches wasserdampfdurchlässig sind.

4. Selbstschliessendes antiseptisches Pflaster (1) nach Patentanspruch 1 oder 2, **dadurch gekennzeichnet, dass** die Trägerschicht (2) stabilisierende Eigenschaften aufweist und weniger dehnbar ist als die Verschlussschicht (5).

5. Selbstschliessendes antiseptisches Pflaster (1) nach einem der Patentansprüche 1 bis 4, **dadurch gekennzeichnet, dass** die Trägerschicht (2) flüssigkeitsabsorbierend ist.

6. Selbstschliessendes antiseptisches Pflaster (1) nach einem der Patentansprüche 1 bis 5, **dadurch gekennzeichnet, dass** die Kleberschicht (4) keine hautkleberspezifischen Eigenschaften aufzuweisen braucht

7. Selbstschliessendes antiseptisches Pflaster (1) nach einem der Patentansprüche 1 bis 6, **dadurch gekennzeichnet, dass** die Kleberschicht (4) in einem für Nadeleinstiche vorgesehenen Bereich eine kleberfreie Aussparung aufweist.

8. Selbstschliessendes antiseptisches Pflaster (1) nach einem der Patentansprüche 1 bis 7, **dadurch gekennzeichnet, dass** die Kleberschicht (4) einen Trägerfilm aufweist der beidseitig mit Kleber versehen ist.

9. Selbstschliessendes antiseptisches Pflaster (1) nach einem der Patentansprüche 1 bis 8, **dadurch gekennzeichnet, dass** die Hautkleberschicht (3) durch Beigabe entsprechender Wirkstoffe eine oder mehrere der Eigenschaften aus der folgenden Gruppe von Eigenschaften aufweist:
- antiseptische Eigenschaften,
- antiallergische Eigenschaften,
- analgetische Eigenschaften.

10. Selbstschliessendes antiseptisches Pflaster (1) nach einem der Patentansprüche 1 bis 9, **dadurch gekennzeichnet, dass** die Verschlussschicht (5) zumindest teilweise aus Naturkautschuk, Kunstkautschuk, Gummi, Latex, Silikon, Hydrogel, polymerem Kunststoff oder einer Kombination dieser Materialien besteht und selbstverschliessende Eigenschaften aufweist.

11. Selbstschliessendes antiseptisches Pflaster (1) nach einem der Patentansprüche 1 bis 10, **dadurch gekennzeichnet, dass** die Verschlussschicht (5) vorzugsweise aus kleberschichtseitig plasma-, corona-, nasschemisch- oder sonstig vorbehandeltem Silikon ist.

12. Selbstschliessendes antiseptisches Pflaster (1) nach Patentanspruch 11, **dadurch gekennzeichnet, dass** das antiseptische Pflaster (1) eine Gesamtdicke im Bereich von 1 - 5 mm aufweist.

13. Selbstschliessendes antiseptisches Pflaster (1) nach Patentanspruch 4, **dadurch gekennzeichnet, dass** die Trägerschicht (2) vorzugsweise aus Polyurethan ist und eine Dicke im Bereich von 0.01 - 0.05 mm aufweist.

## Claims

1. Self-sealing antiseptic plaster (1) for puncturing blood vessels, muscular tissue, tissue, skin, organs and bone marrow, which can be pierced by cannulas, needles or similar and which is transparent in a partial area, comprising
- a support layer (2),
- a transparent and pierceable sealing layer (5) that is applied to the support layer (2) and consists of a material having an elastic restoring force,
- a cutaneous adhesive layer (3) applied to the underside U of the support layer (2),
**characterised in that**
- the support layer (2) has no recesses,
- an adhesive layer (4) is provided between the support layer (2) and the sealing layer (5) and
- the support layer (2), the sealing layer (5), the adhesive layer (4) and the cutaneous adhesive layer (3) are transparent or approximately transparent in the entire area or at least in a superposed partial area.

2. The self-sealing antiseptic plaster (1) according to claim 1, **characterised in that** the support layer (2) and the sealing layer (5) are made of thin and soft materials so that the point to be punctured can be felt through the self-sealing antiseptic plaster (1).

3. The self-sealing antiseptic plaster (1) according to claim 1 or 2, **characterised in that** the support layer (2) and/or the sealing layer (5) are permeable to water vapour outside a piercing area.

4. The self-sealing antiseptic plaster (1) according to claim 1 or 2, **characterised in that** the support layer (2) has stabilising properties and is less expandable that the sealing layer (5).

5. The self-sealing antiseptic plaster (1) according to any one of claims 1 to 4, **characterised in that** the support layer (2) is liquid-absorbing.

6. The self-sealing antiseptic plaster (1) according to any one of claims 1 to 5, **characterised in that** the adhesive layer (4) does not need to have any cutaneous adhesive specific properties.

7. The self-sealing antiseptic plaster (1) according to any one of claims 1 to 6, **characterised in that** the adhesive layer (4) has an adhesive-free recess in an area provided for needle injections.

8. The self-sealing antiseptic plaster (1) according to any one of claims 1 to 7, **characterised in that** the adhesive layer (4) has a support film which is provided with adhesive on both sides.

9. The self-sealing antiseptic plaster (1) according to any one of claims 1 to 8, **characterised in that** the cutaneous adhesive layer (3) has one or more of the properties from the following group of properties as a result of the addition of appropriate active substances:
- antiseptic properties,
- antiallergic properties,
- analgesic properties.

10. The self-sealing antiseptic plaster (1) according to any one of claims 1 to 9, **characterised in that** the sealing layer (5) consists at least partially of natural rubber, synthetic rubber, vulcanised rubber, latex, silicone, hydrogel, polymeric plastic or a combination of these materials and exhibits self-sealing properties.

11. The self-sealing antiseptic plaster (1) according to any one of claims 1 to 10, **characterised in that** the sealing layer (5) is preferably silicone which has been subjected to plasma, corona, wet-chemical or other pre-treatment on the adhesive layer side.

12. The self-sealing antiseptic plaster (1) according to claim 11, **characterised in that** the antiseptic plaster (1) has a total thickness in the range of 1-5 mm.

13. The self-sealing antiseptic plaster (1) according to claim 4, **characterised in that** the support layer (2) preferably consists of polyurethane and has a thickness in the range of 0.01-0.05 mm.

## Revendications

1. Pansement (1) antiseptique à autofermeture, pour ponctionner des vaisseaux sanguins, des tissus musculaires, des tissus, la peau, des organes ou de la moelle osseuse, perforable par des canules, des aiguilles ou similaires et transparent sur une zone partielle, avec
- une couche support (2),
- une couche de terminaison (5) transparente et perforable en une matière à force de rappel élastique, montée sur la couche support (2),
- une couche d'adhésif cutané (3) placée sur la face inférieure U de la couche support (2),
**caractérisé en ce que**
- la couche support (2) ne présente aucun évidement,
- une couche d'adhésif (4) est présente entre la couche support (2) et la couche de terminaison (5), et
- la couche support (2), la couche de terminaison (5), la couche d'adhésif (4) et la couche d'adhésif cutané (3) sont transparentes ou au moins partiellement transparentes sur toute la zone ou au moins sur une zone partielle superposée.

2. Pansement (1) antiseptique à autofermeture selon la revendication 1, **caractérisé en ce que** la couche support (2) et la couche de terminaison (5), sont fabriquées en une matière mince et souple, pour que la zone à ponctionner soit palpable à travers le pansement (1) antiseptique à autofermeture.

3. Pansement (1) antiseptique à autofermeture selon la revendication 1 ou 2, **caractérisé en ce que** la couche support (2) et/ou la couche de terminaison (5), sont perméables à la vapeur d'eau, à l'extérieur d'une zone de perforation.

4. Pansement (1) antiseptique à autofermeture selon la revendication 1 ou 2, **caractérisé en ce que** la couche support (2) a des propriétés stabilisatrices et est moins extensible que la couche de terminaison (5).

5. Pansement (1) antiseptique à autofermeture selon l'une quelconque des revendications 1 à 4, **caractérisé en ce que** la couche support (2) absorbe les liquides.

6. Pansement (1) antiseptique à autofermeture selon l'une quelconque des revendications 1 à 5, **caractérisée en ce que** la couche d'adhésif (4) n'a pas besoin de présenter des propriétés spécifiques d'adhésif cutané.

7. Pansement (1) antiseptique à autofermeture selon l'une quelconque des revendications 1 à 6, **caractérisé en ce que** la couche d'adhésif (4) présente dans une zone prévue pour les perforations par aiguilles une échancrure exempte d'adhésif.

8. Pansement (1) antiseptique à autofermeture selon l'une quelconque des revendications 1 à 7,
**caractérisé en ce que** la couche d'adhésif (4) comporte un film support, qui sur les deux faces est muni d'adhésif.

9. Pansement (1) antiseptique à autofermeture selon l'une quelconque des revendications 1 à 8, **caractérisé en ce que**, par ajout de principes actifs correspondants, la couche d'adhésif cutané (3) présente l'une ou plusieurs des propriétés du groupe de propriétés suivant :
- propriétés antiseptiques,
- propriétés antiallergiques,
- propriétés analgésiques.

10. Pansement (1) antiseptique à autofermeture selon l'une quelconque des revendications 1 à 9, **caractérisé en ce que** la couche de terminaison (5), consiste au moins partiellement en caoutchouc naturel, en caoutchouc artificiel, en gomme, en latex, en silicone, en matière plastique polymère ou en une association desdites matières et présente des propriétés d'autofermeture.

11. Pansement (1) antiseptique à autofermeture selon l'une quelconque des revendications 1 à 10, **caractérisé en ce que** la couche de terminaison (5) est de préférence une silicone prétraitée de préférence côté couche adhésive par traitement plasma, corona, chimique humide ou un autre traitement.

12. Pansement (1) antiseptique à autofermeture selon la revendication 11, **caractérisé en ce que** le pansement (1) antiseptique à autofermeture a une épaisseur totale de 1 à 5 mm.

13. Pansement (1) antiseptique à autofermeture selon la revendication 4, **caractérisé en ce que** la couche support (2) est de préférence en polyuréthane et a une épaisseur de l'ordre de 0.01 à 0.05 mm.
